**Europäisches Patentamt**

**European Patent Office**

**Office européen des brevets**

(19)

(11) Veröffentlichungsnummer: **0 009 590**
B1

(12) **EUROPÄISCHE PATENTSCHRIFT**

(45) Veröffentlichungstag der Patentschrift: **24.02.82**

(21) Anmeldenummer: **79103040.6**

(22) Anmeldetag: **20.08.79**

(51) Int. Cl.³: **C 07 C 85/06, C 07 C 85/08**

(54) Verfahren zur Herstellung von Aminen.

(30) Priorität: **01.09.78 DE 2838184**

(43) Veröffentlichungstag der Anmeldung:
**16.04.80 Patentblatt 80/8**

(45) Bekanntmachung des Hinweises auf die Patenterteilung:
**24.02.82 Patentblatt 82/8**

(84) Benannte Vertragsstaaten:
**BE CH DE FR GB IT NL SE**

(56) Entgegenhaltungen:
**DE - A1 - 2 445 303**
**DE - A1 - 2 709 864**
**DE - C - 637 731**
**DE - C - 881 657**
**INDUSTRIAL AND ENGINEERING CHEMISTRY,**
**Product Research and Development, Band 16,**
**Nr. 3, 1977**
**A. BALKER et al. "Catalytic Aminiation of Long**
**Chain Aliphatic Alcohols" Seiten 261 bis 266**

(73) Patentinhaber: **BASF Aktiengesellschaft**
**Carl-Bosch-Strasse 38**
**D-6700 Ludwigshafen (DE)**

(72) Erfinder: **Toussaint, Herbert, Dr.**
**Knietschstrasse 11**
**D-6710 Frankenthal (DE)**
Erfinder: **Schroeder, Wolfgang, Dr.**
**Seebacher Strasse 51**
**D-6702 Bad Duerkheim (DE)**
Erfinder: **Franzischka, Wolfgang, Dr. Dipl.-Ing.**
**Kranichstrasse 11**
**D-6700 Ludwigshafen (DE)**

## Verfahren zur Herstellung von Aminen

Die Erfindung betrifft ein Verfahren zur N-Alkylierung von Aminen, d.h. zur Herstellung sekundärer oder tertiärer Amine durch Umsetzung primärer bzw. sekundärer Amine mit Alkoholen oder Carbonylverbindungen in der Gasphase unter hydrierenden Bedingungen.

Sekundäre und tertiäre Amine finden vielfältige Verwendung, z.B. als Zwischenprodukte und Säurefänger bei bestimmten Synthesen, als Katalysatoren bei der Herstellung von Polyurethanen und als Bakterizide.

Für die Herstellung substituierter Amine durch Alkylierung unvollständig substituierter Amine sind viele Verfahren vorgeschlagen worden, die sich in Gruppen einteilen lassen:

Eine Gruppe ver Verfahren benutzt als Katalysatoren Übergangsmetalle der 8. Gruppe des Periodensystems wie Nickel, insbesondere Raney-Nickel in Gegenwart von Wasserstoff; gelegentlich werden auch die Platinmetalle angegeben. Die Reaktionspartner liegen im allgemeinen in flüssiger Phase vor, seltener in der Gasphase; hierbei wird in vielen Fällen bei atmosphärischem Druck gearbeitet.

Eine andere Gruppe verwendet Katalysatoren vom Typ des Kupfer-Chromoxids (Kupferchromit) nach ADKINS. Dabei wird gewöhnlich ein Druckbereich von 10 bis 25 bar, manchmal bis 200 bar und mehr angewendet, so daß die Reaktionsteilnehmer stets flüssig vorliegen.

Aus der DE—PS 881 657 ist auch ein Verfahren bekannt, bei dem die Reaktionspartner gasförmig zur Reaktion gebracht werden. Hier wird bei 10 bis 15 bar und bei einer relativ hohen Temperatur (zwischen 240 und 300°C) gearbeitet.

Eine dritte Gruppe von Verfahren verwendet als Katalysator ein Oxid mit dehydratisierenden Eigenschaften, z.B. $Al_2O_3$ oder $SiO_2$. Hier werden zum Teil sehr hohe Temperaturen (bis 400°C) und oft auch hohe Drucke (bis 200 bar) angewandt. Nur in Ausnahmefällen gelingt ein derartiges Verfahren bei gewöhnlichem Druck und in der Gasphase (vergleiche z.B. DE—PS 637 731; US—PS 338 4667.

Schon aus der Tatsache der verschiedenen Verfahrensvorschläge läßt sich schließen, daß diese Verfahren sämtlich irgendwelche Nachteile aufweisen:

Die Elemente der 8. Gruppe können wegen ihrer hohen hydrierenden Aktivität schon vorhandene Alkylgruppen vom Stickstoffatom des Amins abspalten und dadurch die Bildung sekundärer, oft sogar primärer Amine, sowie (durch Umalkylierung) anderer als der gewünschten Amine begünstigen.

Die übrigen Verfahren arbeiten gewöhnlich bei hohem Druck; das bedeutet erhöhten Energie — und Materialaufwand und außerdem — im Störungsfall — eine gewisse Gefährdung.

Aufgabe der Erfindung ist daher die Schaffung eines Verfahrens zur N-Alkylierung von Aminen durch Umsetzung der Amine mit Alkoholen oder Carbonylverbindungen an einem Kupferkatalysator unter hydrierenden Bedingungen in der Gasphase bei einem Druck von weniger als 10 bar und einer Temperatur unter 300°C, das einfach arbeitet und mit billigen Katalysatoren auskommt sowie eine hohe Ausbeute erzielt.

Es wurde gefunden, daß man die Umsetzung an einem Kupferkatalysator vornimmt, der in üblicher Weise durch Fällung von verdünnten oder mäßig konzentrierte Lösungen von Kupfer- und Aluminium-salzen mit Alkalicarbonat bei pH 8—10 Zersetzung der erhaltenen Niederschläge vor oder nach entsprechenden Formung bei einer Temperatur von 350—600°C Reduktion erhalten worden ist.

Als Kupferkatalysatoren werden die in der DE—OS 24 45 303 beschriebenen verwendet. Sie können als amorphe Produkte der thermischen Zersetzung und Reduktion basischer Kupfer-Aluminium-Carbonate angesehen und dadurch erhalten werden, daß man verdünnte oder mäßig konzentrierte, zweckmäßig unter 3-molare Löslungen von Kupfer- und Aluminiumsalzen mit Alkalicarbonat bei pH 8—10 fällt und die erhaltenen Niederschläge vor oder nach entsprechender Formung bei einer Temperatur von 350 bis 600°C zersetzt. Auf die in der zuletzt genannten Offenlegungsschrift niedergelegte Beschreibung wird hierzu verwiesen. Nach üblicher Reduzierung erhält man hochaktive, für das vorliegende Verfahren bestens geeignete Katalysatoren.

Das Verfahren ist auf die Umsetzung unvollständig alkylierter aliphatischer und aromatischer Amine gleichermaßen anwendbar. Diese Amine können vor allem mit einwertigen aliphatischen Alkoholen von Methanol bis etwa zum Tetradecanol bei atmosphärischem Druck mit einem primären oder sekundären Amin in Anwesenheit von Wasserstoff umgesetzt werden, bei nahezu quantitativer Ausbeute. Vielfach ebenso wirtschaftlich ist die Verwendung entsprechender Aldehyde und Ketone; sie besitzen im allgemeinen Siedepunkte von bis zu 250°C, bei atmosphärischem Druck.

Umalkylierung und Bildung von sekundären oder primären Aminen aus tertiären bzw. sekundären Aminen werden nur in geringem Ausmaß oder gar nicht beobachtet. Die Umsetzungen können selbst in Glasapparaten vorgenommen werden. Wird die Anlage aus irgendeinem Grund undicht, so gelangen nur geringe Mengen Amin in die Umgebung; im übrigen ist die Wahrscheinlichkeit, daß überhaupt Undichtigkeiten auftreten erheblich geringer als bei Druckprozessen.

Das Verfahren kann wie folgt ausgeführt werden: Als Reaktor verwendet man z.B. ein senkrecht angeordnetes zylindrisches Gefäß, bevorzugt ein senkrecht angeordnetes Rohrbündel mit üblichen Einrichtungen zur Kühlung und Heizung.

Es wird gefüllt mit dem erfindungsgemäßen Katalysator in einer technisch üblichen Formgebung, z.B. etwa 3 bis 10 mm großen zylindrischen Tabletten. Die Zylindergrundflächen können eben, konkav oder konvex gewölbt sein. Von der Zylinderform abweichende Katalysatorkörnungen, z.B. Kugeln oder längliche Strangpreßlinge können ebenfalls verwendet werden. Der Katalysator kann sowohl in vorreduzierten, passiviertem Zustand eingefüllt werden als auch im oxidischen Zustand. Im ersten Fall wird durch eine kurze Behandlung mit einem Stickstoff-Wasserstoffgemisch bei 100 bis 150°C reaktiviert, im zweiten Fall wird die gesamte Reduktion im Reaktor vorgenommen. Danach wird die erforderliche Temperatur eingestellt; im allgemeinen ist dies eine Temperatur zwischen 100 und 250°C. Gleichzeitig wird der während der Umsetzung benötigte Wasserstoffstrom eingestellt, der vorzugsweise im Kreis gefördert wird und den Reaktor aufwärts oder abwärts durchströmen kann. Seine Menge liegt im allgemeinen im Bereich von 100 bis 2000 effektiven Volumeneinheiten Wasserstoff je Volumeneinheit des Katalysators und Stune. Ist der Wasserstoffpartialdruck zu gering, so können Nebenprodukte entstehen und auf die Dauer kann die Aktivität des Katalysators leiden. In den meisten Fällen ist ein Wasserstoffpartialdruck im Bereich um 0,3 bis 0,8 bar ausreichend, wobei die Umsetzung bei insgesamt atmosphärischem Druck möglich ist. Reicht ein Partial-druck von 0,7 bis 0,8 bar nicht aus, so kann eine geringfügige Erhöhung des Gesamtdruckes nötig werden. Das Verfahren ist bei höheren Drucken von weniger als 10 bar funktionsfähig.

Der Wasserstoffstrom wird zweckmäßig zunächst in einem Vorheizer auf eine Temperatur von etwa 200 bis 400°C gebracht und dann in der Weise zur Verdampfung der Reaktionsteilnehmer benutzt, daß diese flüssig einem Verdampfer zugeführt werden, wo sie vom erhitzten Wasserstoff mitgenommen werden.

Das so entstandene Gemisch durchströmt den Reaktor, wird in einem System von Wärmetauschern und Kühlern gegebenenfalls bis auf etwa —10°C gekühlt und notfalls zur Abscheidung kondensierbarer Anteile noch verdichtet. Von hier aus kehrt der Wasserstoff zum Vorheizer zurück.

Besonders wenn man mit einem Aminüberschuß arbeitet — er kann z.B. bis 20 Mol betragen — enthält das den Reaktor verlassende Gemisch außber dem Reaktionsprodukt, überschüssigem Wasserstoff und Wasser noch eine entsprechende Menge des ursprünglichen Amins, während Alkohol bzw. Carbonylverbindungen im allgemeinen umgesetzt worden sind.

Die Trennung dieses Stoffgemisches erfordert zusätzlich zur Abkühlung in der Regel also einen Destillationsschritt zur Abtrennung des nicht umgesetzten Amins sowie ein Verfahren zur Abtrennung des Wassers bzw. Trocknung.

Dies kann ebenfalls durch Destillieren, aber auch z.B. durch Behandeln mit konzentrierter Natronlauge geschehen.

Die Umsetzung mit einem Überschuß an Alkohol bzw. Carbonyl-/-verbindung ist ebenfalls möglich, wobei die Entscheidung, in dieser oder jener Weise zu verfahren, im allgemeinen von wirtschaftlichen Gesichtsprunkten bestimmt wird. Falls die Reaktionsprodukte einen hohen Siedepunkt und das nicht umgesetzte Amin einen niedrigen Siedepunkt haben, was vor allen Dingen in den Fällen zu beobachten ist, wo man mit einem langkettigen Alkohol oder einer langkettigen Carbonylverbindung umgesetzt hat, so kann das nicht umgesetzte leicht flüchtige Amin gemeinsam mit dem Wasserstoff gasförmig im Kreis gefördert und sein Anteil lediglich im Maß der Umsetzung ergänzt werden.

Das Verfahren ist auch aus diesem Grunde besonders geeignet zur Umsetzung mit solchen Alkoholen bzw. Carbonylverbindungen, die eine hohe Siedetemperatur, die unter Umständen auch oberhalb der Reaktionstemperatur liegen kann, besitzen. Besonders in diesen Fällen ist es günstig die Verdampfung dieser hochsiedenden Verbindungen mit Hilfe des im Kreis geführten Gas(gemisches) vorzunehmen, d.h. das Kreisgas im einfachsten Falle damit zu sättigen. Auch in anderen Fällen erleichtert dieses Vorgehen die Verdampfung, wie weiter oben bereits ausgeführt wurde.

Beispiel 1
Umsetzung von Dodecylalkohol mit Dimethylamin (vgl. Figur)

Der Reaktor besteht aus einem zylindrischen, senkrecht stehenden und durch einen umlaufenden organischen Wärmeträger beheizbaren Gefäß (8), das mit einem Katalysator in Form von Zylindern von 3 mm Höhe und 3 mm Durchmesser gefüllt ist; der Katalysator war nach der Vorschrift des Beispiels 1 der DE—OS 24 45 303 hergestellt worden. Man reduziert bei 120 bis 140°C, indem man durch die Lietung (3) zunächst mit Stickstoff spült, dem dann 3 bis 5 Volumenprozent Wasserstoff zugesetzt werden. Wenn die Wasserabscheidung nachläßt, wird die Temperatur allmählich bis auf 200°C erhöht. Wenn die Wasserbildung aufgehört hat, wird der Stickstoff ersetzt durch Wasserstoff. Man stellt eine Gasgeschwindigkeit von 570 Volumenteilen (STP) pro Stunde bezogen auf das vom Katalysator eingenommene Reaktorvolumen ein. Im Wärmetauscher (6) wird der Wasserstoff auf ca. 250°C vorgeheizt. Dem Wasserstoffstrom werden durch Leitung (2) 28 Volumenteile (gasförmig, STP) Dimethylamin pro Stunde zugesetzt, das im Wärmetauscher (5) auf ca. 250°C vorgeheizt worden war.

Für die Umsetzung wird handelsüblicher Dodecanol wie 1,3:1 durch die Leitung (1) und graphisch etwa 1,4 (Flächen-)Prozent Anteile kürzerer Retentionszeit enthält. Stündlich wird 0,1 Volumenteil (flüssig) Alkohol entsprechend einem Molverhältnis von Dimethylamin zu Dodecanol wir 1,3:1 durch die Leitung (1) und durch den Wärmetauscher (4) dem Gasgemisch zugeführt und dann dem Verdampfer (7) zugeleitet. Bei ca. 200°C wird vollständig verdampft. Das so erhaltene Gasgemisch tritt mit einem Druck von etwa 1,1 bar in den Reaktor ein. Die Reaktion wird so geleitet, daß die höchste Temperatur 170°C beträgt. Die heißeste Stelle liegt etwa zweischen dem 1. und 2. Viertel der Katalysatorschüttung in Strömungsrichtung. Zum Reaktorausgang hin fällt die Temperatur um etwa 10°C ab. Im Kühler (9) wird das Reaktionsgemisch auf Raumtemperatur gekühlt. Das Kondensat sammelt sich im Abscheider (10). Es hat folgende gaschromatographische Zusammensetzung:

| | | |
|---|---|---|
| Dimethyldodecylamin | : | 97,0% |
| Dimethylamin | : | 0,6% |
| Monomethyldodecylamin | : | 0,3% |
| Wasser (nach K. Fischer) | : | 0,9% |
| Sonstige | : | 1,2% |

Nicht umgesetztes Dodecanol und andere schwersiedende Bestandteile wurden nicht gefunden.

### Beispiel 2
### Umsetzung von Piperidin mit Methanol

Es wird die in Beispiel 1 beschriebene Anlage benutzt. Bei einer maximalen Reaktionstemperatur von 180°C wird eine Wasserstoffmenge von 120 Volumenteilen (STP) pro Volumenteil Katalysator und Stunde durchgesetzt. Die Vorgabetemperatur im Verdampfer 7 war 250°C. Dem Wasserstoffstrom wurde ein Gemisch aus Piperidin und Methanol im Molverhältnis 1:4 zugeführt, dessen Menge im flüssigen Zustand 0,2 Volumenteilen entsprach. Das erhaltene Kondensat hat folgende gaschromatographische Zusammensetzung:

| | | |
|---|---|---|
| N-Methylpiperidin | : | 79,3% |
| Piperidin | : | — |
| Methanol | : | 19,4% |
| Rest | : | 1,3% |

### Beispiel 3
### Umsetzung von Cyclohexanon mit Dimethylamin

Es wird die in Beispiel 1 beschriebene Anlage benutzt. Die mittlere Reaktionstemperatur beträgt 140°C, der Wasserstoffstrom war 60 Volumenteile (STP) je Stunde und Volumenteil Katalysator. Die Temperatur im Wärmetauscher (6) und Verdampfer (7) liegt ebenfalls bei 140°C. Über die Leitungen (2) und den Wärmetauscher (5) werden 20 Volumenteile (STP) pro Stunde Dimethylamin zugeführt. Durch die Leitungen (1) und den Wärmetauscher (4) wird dem Gasstrom Cyklohexanon in einer Menge von 0,03 Volumenteilen (flüssig) pro Stunde beigemischt. Die höchste gemessene Temperatur im Reaktor beträgt 160°C. Das Reaktionsgemisch wird auf Raumtemperatur abgekühlt und hat folgende gaschromatographische Zusammensetzung (wasserfrei):

| | | |
|---|---|---|
| Dimethylcyclohexylamin | : | 89% |
| Dimethylamin | : | 8% |
| Cyclohexanol | : | 1% |
| Rest | : | 2% |

### Beispiel 4
### Umsetzung von Äthanol mit Dimethylamin Man verfährt wie in Beispiel 1 beschrieben. Über den Wärmetauscher (6) und dem Verdampfer (7), deren Temperatur auf einheitlich 150°C eingestellt worden sind werden stündlich 60 Volumenteile Wasserstoff je Volumenteil Katalysator zugeführt. 20 Volumenteile gasförmiges Methylamin werden nach dem passieren des Wärmetauschers (5), wo ebenfalls eine Temperatur von 150°C eingestellt wird, dem Wasserstoffstrom beigemischt und über den Wärmetauscher (4) 0,1 Volumenteil flüssiges Äthanol je Stunde dem Gasstrom zugefügt, die im Verdampfer (7) vollständig verdampfen. Das Reaktionsgemisch wird nach dem Verlassen des Reaktors in 2 Stufen gekühlt; die bei Raumtemperatur erhaltene Fraktion hat (wasserfrei gerechnet) folgende Zusammensetzung:

| | | |
|---|---|---|
| Dimethyläthylamin | : | 49% |
| Äthanol | : | 48% |
| Rest | : | 2% |

In einem zweiten Kühler (12) wird das Restgas auf —40°C gekühlt, wodurch im Behälter (13) ein Tieftemperaturkondensat mit folgender Zusammensetzung anfällt.

| | | |
|---|---|---|
| Dimethyläthylamin | : | 90% |
| Äthanol | : | 6% |
| Dimethylamin | : | 2% |
| Rest | : | 2% |

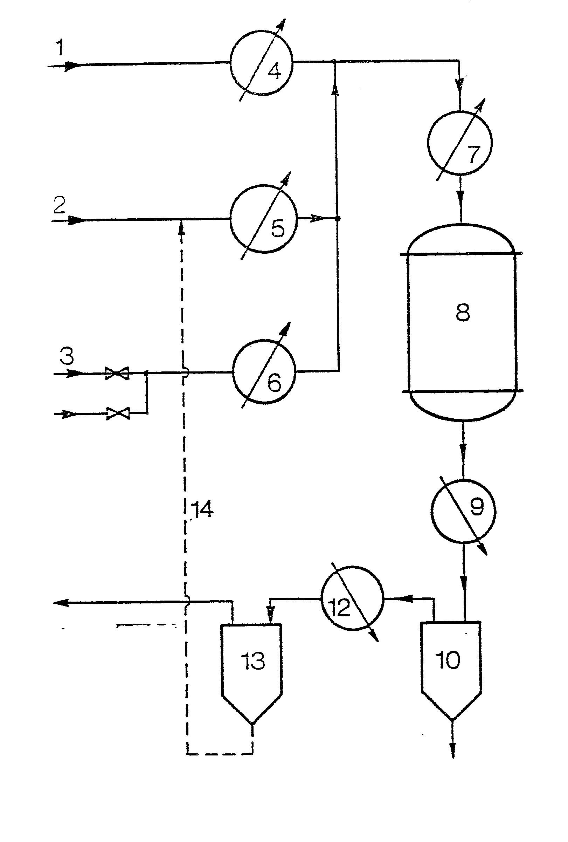

Dieses Kondensat wird in einer Menge von 14%, bezogen auf die gesamte kondensierbare Menge an Bestandteilen zugebildet und erneut dem Reaktor zugeführt (symbolisiert durch die nicht ausgezogene Linie (14) in der Figur).

Beispiel 5

Zur Gewinnung von N-n-Propylanilin führt man dem im Beispiel 1 beschriebenen Reaktor stündlich ein Gemisch aus 0,35 Volumenteilen (flüssig) Anilin und Propanol im Molverhältnis 1:1,1 zu, das von dem auf 200°C vorgeheizten Wasserstoff (120 Volumenteile; STP) im Verdampfer aufgenommen wird. Der Druck am Reaktoreingang beträgt 1,1 bar; man läßt 190°C im Reaktor nich übersteigen. Das im Abscheider gesammelte Kondensat bildet eine wäßrige und eine organische Schicht; die letzte besteht aus 89% N-n-Propylanilin, je 4% Propanol und Anilin und 3% sonstigen Bestandteilen.

Beispiel 6

In analoger Weise wie in Beispiel 5 wird N-Äthyltoluidin aus o-Toluidin und Äthanol hergestellt. Die Wasserstoffmenge beträgt 150 Volumenteile (STP) und das Gemisch der Ausgangstoffe 0,5 Volumenteile (flüssig. Man erhält ein Gemisch aus 80% N-Äthyl-o-touidin, 2% N,N-Diäthyl-o-Toluidin, 13% o-Toluidin, 4% Äthanol und 1% sonstigen Bestandteilen.

Weitere Beispiele

Mit ähnlich gutem Erfolg wie vorstehend beschrieben, konnte z.B. N,N'-Dimethylpiperazin aus Methanol und Piperazin; M-Laurylmorpholin aus Morpholin und Laurylalkohol; N,N-Di-n-butylanilin aus N-Butyraldehyd und N-n-Butylanilin erhalten werden. Bei Umsetzungen mit Methanol sollten 170°C nicht überschritten werden, um dessen Zerfall in CO+H$_2$ zu vermeiden.

Patentanspruch

Verfahren zur N-Alkylierung von Aminen durch Umsetzung der Amine mit Alkoholen oder Carbonylverbindungen an einem Kupferkatalysator unter hydrierenden Bedingungen in der Gasphase bei einem Druck von weniger als 10 bar und einer Temperatur unter 300°C, dadurch gekennzeichnet, daß man die Umsetzung an einem Kupferkatalysator vornimmt, der in üblicher Weise durch Fällung von verdünnten oder mäßig konzentrierte Kupfer — und Aluminiumsalz lösungen mit Alkalicarbonat bei pH 8—10 Zersetzung der erhaltenen Niederschläge vor oder nach einer entsprechenden Formung bei einer Temperatur von 350—600°C und Reduction erhalten worden its.

Revendication

Procédé pour la N-alcoylation d'amines par réaction des amines en phase gazeuse, dans des conditions hydrogénantes, sous une pression inférieure à 10 bars et à une température inférieure à 300°C, en présence d'un catalyseur au cuivre avec des alcools ou des composés carbonylés, caractérisé en ce que la réaction est effectuée en présence d'un catalyseur au cuivre, préparé de manière connue en soi par précipitation de sels de cuivre et d'aluminium en solution diluée ou modérément concentrée par un carbonate de métal alcalin à un pH entre 8 et 10, suivie de la décomposition des précipités formés, avant ou après leur formage, à une température comprise entre 350 et 600°C, et d'une réduction.

Claim

A process for N-alkylating an amine by reacting it with an alcohol or carbonyl compound in the gas phase over a copper catalyst under hydrogenating conditions at a pressure of less than 10 bar and a temperature below 300°C, characterized in that the reaction is carried out over a copper catalyst which has been obtained in the conventional manner by precipitating a dilute or moderately concentrated solution of salts of copper and aluminium with an alkali metal carbonate at pH 8—10, decomposing the resulting precipitate, before or after appropriate molding, at 350—600°C, and reducing the product.